# EUROPEAN PATENT APPLICATION

(11) **EP 4 040 320 A1**
(43) Date of publication of application: **10.08.2022**
(21) Application number: 21200683.7
(22) Date of filing: 04.10.2021
(51) Int. Cl.: G06F 21/62, A61B 5/11, H04W 84/18, G06N 20/00, H04W 12/02, H04W 12/33

(54) **ON-DEVICE ACTIVITY RECOGNITION**

(30) Priority: 05.02.2021 US 202117169194
(71) Applicant: Google LLC, Mountain View, CA 94043 (US)
(72) Inventor: Phillips, Justin Paul, London (GB); Harle, Robert Keith, London (GB); Nashikkar, Pranav, London (GB); Wilson, Alex, London (GB); Alewijnse, Sander, London (GB)
(74) Representative: Anderson, Oliver Ben

(57) **Abstract**

A computing device may receive motion data generated by one or more motion sensors that correspond to movement sensed by the one or more motion sensors. The computing device may perform, using one or more neural networks trained with differential privacy, on-device activity recognition to recognize a physical activity that corresponds to the motion data. The computing device may, in response to recognizing the physical activity that corresponds to the motion data, perform an operation associated with the physical activity.

## Description

### BACKGROUND

Some mobile and wearable computing devices may track user activity to assist users in maintaining healthier and more active lifestyles. For instance, a mobile computing device may include one or more sensor components, which provide sensor data that may be indicative of a user engaging in a physical activity. The mobile computing device may send the data provided by the one or more sensor components off-device, such as to a cloud computing system, for processing in order to identify the physical activity being performed by the user based on the sensor data.

### SUMMARY

In general, techniques of this disclosure are directed to performing on-device recognition of activities in which the user of a computing device is engaged using one or more neural networks that are trained with differential privacy. The computing device may recognize the activity of the user based on the sensor data provided by the one or more sensor components without sending the sensor data to an external computing system (e.g., to the cloud). Instead, the computing device may use one or more neural networks trained to perform activity recognition to perform on-device activity recognition based on the sensor data provided by the one or more sensor components.

The one or more neural networks may be trained off-device to perform activity recognition in ways that use fewer computing resources (e.g., using fewer processing cycles and less memory) compared with neural networks that perform server-side activity recognition, so that the computing device may be able to use one or more neural networks to perform on-device activity recognition. By using one or more neural networks to perform on-device activity recognition, the computing device may be able to accurately recognize the activity of the user without having to send and receive data to an external computing system that performs server-side activity recognition. Instead, the sensor data provided by the one or more sensor components may be kept on the computing device, thereby preserving the privacy of the user. In addition, performing on-device activity recognition may improve performance of the computing device, as further described below.

Training one or more neural networks with differential privacy adds noise to hide individual examples in the training dataset of the one or more neural networks. By training the one or more neural networks with differential privacy, the one or more neural networks offer strong mathematical guarantees that the one or more neural networks do not learn or remember the details about any specific user whose data was used to train the one or more neural networks. For example, differential privacy may prevent a malicious actor from accurately determining whether a specific piece of data was used during training of the one or more neural networks, thereby preserving the privacy of users whose data was used to train the one or more neural networks. As such, training the one or more neural networks with differential privacy may enable the one or more neural networks to be trained based on free living data from users by preserving the privacy of users whose data was used to train the one or more neural networks.

In some examples, a method includes receiving, by a computing device, motion data generated by one or more motion sensors that correspond to movement sensed by the one or more motion sensors; perform, by the computing device using one or more neural networks trained with differential privacy, on-device activity recognition to recognize a physical activity that corresponds to the motion data; and in response to recognizing the physical activity that corresponds to the motion data, performing, by the computing device, an operation associated with the physical activity.

In some examples, a computing device includes a memory; and one or more processors configured to: receive motion data generated by one or more motion sensors that correspond to movement sensed by the one or more motion sensors; perform, using one or more neural networks trained with differential privacy, on-device activity recognition to recognize a physical activity that corresponds to the motion data; and in response to recognizing the physical activity that corresponds to the motion data, perform an operation associated with the physical activity.

In some examples, a computer-readable storage medium storing instructions that, when executed, cause one or more processors of a computing device to: receive motion data generated by one or more motion sensors that correspond to movement sensed by the one or more motion sensors; perform, using one or more neural networks trained with differential privacy, on-device activity recognition to recognize a physical activity that corresponds to the motion data; and in response to recognizing the physical activity that corresponds to the motion data, perform an operation associated with the physical activity.

In some examples, an apparatus comprises: means for receiving motion data generated by one or more motion sensors that correspond to movement sensed by the one or more motion sensors; means for performing, using one or more neural networks trained with differential privacy, on-device activity recognition to recognize a physical activity that corresponds to the motion data; and means for, in response to recognizing the physical activity that corresponds to the motion data, perform an operation associated with the physical activity.

The details of one or more examples are set forth in the accompanying drawings and the description below. Other features, objects, and advantages of the disclosure will be apparent from the description and drawings, and from the claims.

### BRIEF DESCRIPTION OF DRAWINGS

FIG. 1 is a conceptual diagram illustrating a computing device 110 that may perform on-device recognition of a physical activity using one or more neural networks trained using differential privacy, in accordance with one or more aspects of the present disclosure.
FIG. 2 is a block diagram illustrating further details of a computing device that performs on-device activity recognition using an activity recognition model trained using differential privacy, in accordance with one or more aspects of the present disclosure.
FIGS. 3A-3E are conceptual diagrams illustrating aspects of an example machine-learned model trained using differential privacy according to example implementations of the present disclosure.
FIG. 4 is a flow diagram illustrating example operations of a computing device that performs on-device activity recognition using an activity recognition model trained using differential privacy, in accordance with one or more aspects of the present disclosure.

### DETAILED DESCRIPTION

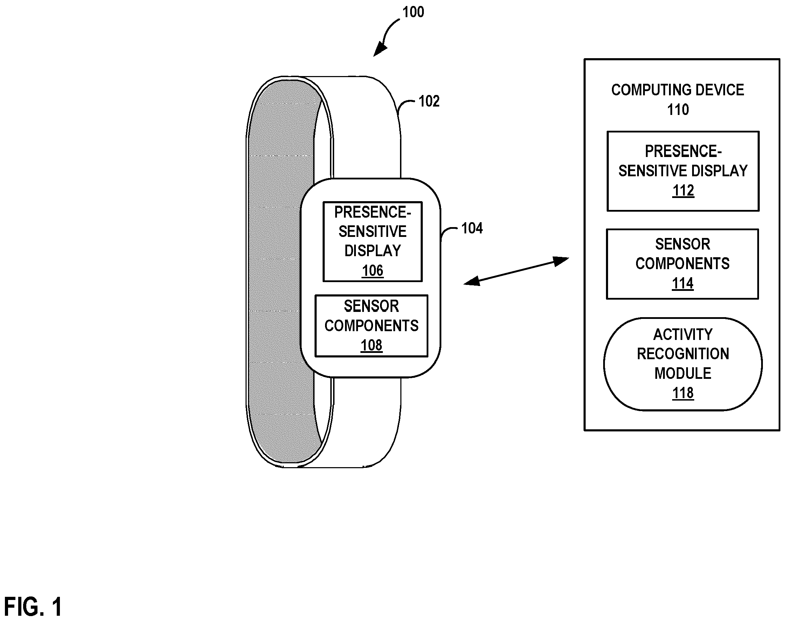

FIG. 1 is a conceptual diagram illustrating a computing device 110 that may perform on-device recognition of a physical activity using one or more neural networks trained using differential privacy, in accordance with one or more aspects of the present disclosure. As shown in FIG. 1, computing device 110 may be a mobile computing device, such as a mobile phone (including a smart phone), a laptop computer, a tablet computer, a wearable computing device, a personal digital assistant (PDA), or any other computing device suitable for detecting an activity of a user. In some examples, computing device 110 may be a wearable computing device such as a computerized watch, a computerized fitness band/tracker, computerized eyewear, computerized headwear, a computerized glove, or any other type of mobile computing device that can attach to and be worn on a person's body or clothing.

In some examples, computing device 110 may include a presence-sensitive display 112. Presence-sensitive display 112 of computing device 110 may function as an input device for computing device 110 and as an output device. Presence-sensitive display 112 may be implemented using various technologies. For instance, presence-sensitive display 112 may function as an input device using a presence-sensitive input component, such as a resistive touchscreen, a surface acoustic wave touchscreen, a capacitive touchscreen, a projective capacitance touchscreen, a pressure-sensitive screen, an acoustic pulse recognition touchscreen, or another presence-sensitive display technology. Presence-sensitive display 112 may function as an output (e.g., display) device using any one or more display components, such as a liquid crystal display (LCD), dot matrix display, light emitting diode (LED) display, microLED, organic light-emitting diode (OLED) display, e-ink, or similar monochrome or color display capable of outputting visible information to a user of computing device 110.

Computing device 110 may also include one or more sensor components 114. In some examples, a sensor component may be an input component that obtains environmental information of an environment that includes computing device 110. A sensor component may be an input component that obtains physiological information of a user of computing device 110. In some examples, a sensor component may be an input component that obtains physical position, movement, and/or location information of computing device 110. For example, sensor components 114 may include, but are not limited to: motion sensors (e.g., accelerometers, gyroscopes, etc.) heart rate sensors, temperature sensors, position sensors, pressure sensors (e.g. a barometer), proximity sensors (e.g., an infrared sensor), ambient light detectors, location sensors (e.g., Global Positioning System sensor), or any other type of sensing component. As further described in this disclosure, activity recognition module 118 may determine one or more physical activities performed by the user of computing device 110 based on sensor data generated by one or more of sensor components 114 and/or one or more sensor components 108 of wearable computing device 100.

In some examples, computing device 110 may be communicatively coupled to one or more wearable computing device 100. For instance, computing device 110 may use one or more communication protocols to send and receive data with wearable computing device 100. In some example examples, communication protocols may include Bluetooth^{®}, Near-Field Communication, WiFi^{®}, or any other suitable communication protocol.

In the example of FIG. 1, wearable computing device 100 is a computerized watch. However, in other examples, wearable computing device 100 may be a computerized fitness band/tracker, computerized eyewear, computerized headwear, a computerized glove, etc. In other examples, wearable computing device 100 may be any type of mobile computing device that can attach to and be worn on a person's body or clothing.

As shown in FIG. 1, in some examples, wearable computing device 100 may include attachment component 102 and electrical housing 104. Housing 104 of wearable computing device 100 includes a physical portion of a wearable computing device that houses a combination of hardware, software, firmware, and/or other electrical components of wearable computing device 100. For example, FIG. 1 shows that within housing 104, wearable computing device 100 may include sensor components 108, and presence-sensitive display 106.

Presence-sensitive display 106 may be a presence-sensitive display as described with respect to presence-sensitive display 112, and sensor components 108 may be sensor components as described with respect to sensor components 114. Housing 104 may also include other hardware components and/or software modules not shown in FIG. 1, such as one or more processors, memories, operating systems, applications, and the like.

Attachment component 102 may include a physical portion of a wearable computing device that comes in contact with a body (e.g., tissue, muscle, skin, hair, clothing, etc.) of a user when the user is wearing wearable computing device 100 (though, in some examples, portions of housing 104 may also come in contact with the body of the user). For example, in cases where wearable computing device 100 is a watch, attachment component 102 may be a watch band that fits around a user's wrist and comes in contact with the skin of the user. In examples where wearable computing device 100 is eyewear or headwear, attachment component 102 may be a portion of the frame of the eyewear or headwear that fits around a user's head, and when wearable computing device 100 is a glove, attachment component 102 may be the material of the glove that conforms to the fingers and hand of the user. In some examples, wearable computing device 100 can be grasped and held from housing 104 and/or attachment component 102.

As shown in FIG. 1, computing device 110 may include activity recognition module 118. Activity recognition module 118 may determine one or more activities of a user based on sensor data generated by one or more of sensor components 114 or, when computing device 110 is communicably coupled to wearable computing device 100, sensor data generated by one or more of sensor components 108 of wearable computing device 100, or sensor data generated by a combination of one or more of sensor components 114 and one or more of sensor components 108. Activities detected by activity recognition module 118 may include but are not limited to: riding on a bicycle, running, remaining still (e.g., sitting or standing), climbing stairs, walking, swimming, yoga, weightlifting, and the like.

Although activity recognition module 118 is described as implemented and operating at computing device 110 for example purposes, wearable computing device 100 may also implement and/or operate an activity recognition module that includes functionality described with respect to activity recognition module 118. In some examples, if the user of computing device 110 is wearing wearable computing device 100, activity recognition module 118 may determine one or more activities of the user based on sensor data generated by one or more sensor components 108 of wearable computing device 100.

In general, if activity recognition module 118 of computing device 110 determines one or more activities of the user based on sensor data generated by one or more sensor components 108 of wearable computing device 100, both computing device 110 and wearable computing device 100 may be under the control of the same user. That is, the same user that wears wearable computing device 100 may be the same user that is using and/or otherwise controlling computing device 110. For example, a user wearing wearable computing device 100 may also be carrying or holding computing device 110 or may have computing device 110 within the physical vicinity (e.g., in the same room) of the user. As such, computing device 110 may not be, for example, a remote computing server or a cloud computing system that communicates with wearable computing device 100 via, for example, the Internet to remotely perform activity recognition based on sensor data generated by one or more sensor components 108 of wearable computing device 100.

In some examples, an activity recognition module implemented and/or operating at wearable computing device 100 may send and receive information to and from activity recognition module 118 via wired or wireless communication. Activity recognition module 118 may use such information received from wearable computing device 100 in accordance with techniques of the disclosure, as though the information were generated locally at computing device 110.

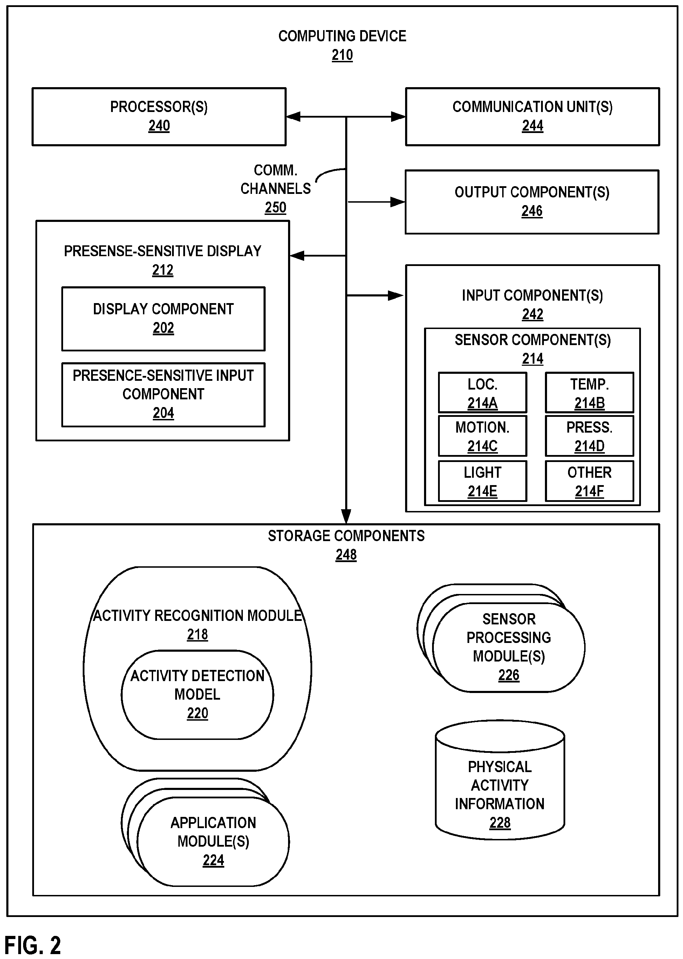

Activity recognition module 118 may receive sensor data that correspond to one or more of sensor components 114 and/or one or more sensor components 108 and determine one or more physical activities that the user is engaged in. In some examples, activity recognition module 118 may receive sensor data from sensor processing modules (e.g., as further described in FIG. 2). Sensor processing modules may provide interfaces between hardware that implement sensor components 114 and modules, such as activity recognition module 118, that further process the sensor data. For example, a sensor processing module may generate sensor data that represent or otherwise correspond to outputs of hardware that implement a particular sensor component. As an example, a sensor processing module for an accelerometer sensor component may generate sensor data that includes acceleration values along different axes of a coordinate system (e.g., x, y, and z axes).

Activity recognition module 118 may perform on-device recognition of the physical activity of the user based on sensor data. That is, activity recognition module 118 may identify the activity of the user without sending information, such as the sensor data, off device, such as to a cloud computing system. Instead, activity recognition module 118 may implement and use one or more neural networks to determine, based on the sensor data, the activity of the user. Examples of sensor data that activity recognition module 118 may use to perform on-device recognition of the physical activity of the user may include motion data generated by one or more motion sensors. As described herein, motion data may include acceleration values along different axes of a coordinate system, generated by one or more multi-axial accelerometers, heartrate data generated by a heartrate sensor, location data generated by a location sensor (e.g., a global positioning system (GPS) sensor), oxygen saturation data (e.g., peripheral oxygen saturation) generated by an oxygen saturation sensor, and the like. As described herein, motion sensors for generating motion data may include such multi-axial accelerometers, heart rate sensors, location sensors, oxygen saturation sensors, gyroscopes, and the like.

In general, one or more neural networks implemented by activity recognition module 118 may include multiple interconnected nodes, and each node may apply one or more functions to a set of input values that correspond to one or more features, and provide one or more corresponding output values. The one or more features may be the sensor data, and the one or more corresponding output values of one or more neural networks may be an indication of the activity of the user that corresponds to the sensor data.

The one or more corresponding output values may, in some examples, include probabilities of activities of the user. Accordingly, activity recognition module 118 may use one or more neural networks to determine probabilities of activities of the user based on the features of the user input, and may determine and output an indication of an activity of the user having the highest probability of being the activity of the user based on the corresponding probabilities.

In some examples, the one or more neural networks may be trained on-device by activity recognition module 118 to more accurately determine the physical activity having the highest probability of being the physical activity of the user based on the features. For instance, one or more neural networks may include one or more learnable parameters or "weights" that are applied to the features. Activity recognition module 118 may adjust these learnable parameters during the training to improve the accuracy with which one or more neural networks determines the physical activity of the user that corresponds to the sensor data and/or for any other suitable purpose, such as to learn sub-features of the way in which the activity is conducted. For example, based on whether the user provides user input to indicate the actual physical activity of the user, activity recognition module 118 may adjust a learnable parameter.

In some examples, the one or more neural networks may be trained off-device and then downloaded to or installed at computing device 110. In particular, the one or more neural networks may be trained using differential privacy. That is, the one or more neural networks may be trained in ways, such as by adding noise in the training data of the one or more neural networks to hide individual examples in the training data, which offer strong mathematical guarantees, also referred to as a privacy guarantee, that the one or more neural networks do not learn or remember the details about any specific user whose data was used to train the one or more neural networks. Training the one or more neural networks using differential privacy thereby preventing malicious actors from accurately determining whether a specific piece of data was used during training of one or more neural networks or how the one or more neural networks were trained, thereby preserving the privacy of users whose data were used to train the one or more neural networks.

The one or more neural networks may be trained using training data that may include sensor data provided by sensor components of computing devices used by a population of users. For example, the training data may include sensor data provided by sensor components of computing devices used by users while performing a variety of physical activities such as riding bicycles, running, remaining still (e.g., not moving), walking, and the like. The resulting trained one or more neural networks may be quantized and compressed so that the one or more neural networks can be installed at a mobile computing device, such as computing device 110, to perform activity recognition. For example, the model weights in the one or more neural networks may be compressed into 8-bit integers for more efficient on-device inference.

Activity recognition module 118 may generate, based on the sensor data and using the one or more neural networks, one or more probabilities, such as a probability distribution, that correspond to one or more physical activities. That is, activity recognition module 118 may, for sensor data at a particular period in time, generate respective probabilities for respective physical activities. Activity recognition module 118 may use sensor data generated by one or more of sensor components 114, one or more of sensor components 108, and/or a combination of any of sensor components 114 and sensor components 108. As an example, activity recognition module 118 may, for sensor data, generate a probability of 0.6 that the user is walking, a probability of 0.2 that the user is running, a probability of 0.2 that the user is riding a bicycle, and a probability of 0.0 that the user is remaining still (i.e., not moving).

Activity recognition module 118 may determine, based at least in part on the one or more probabilities that correspond to one or more physical activities, the physical activity that corresponds to the sensor data. For example, activity recognition module 118 may determine, for the activity having the highest probability out of the one or more probabilities (e.g., the physical activity of walking having a probability of 0.6 in the example described above), whether the corresponding probability satisfies a threshold (e.g., a probability is greater than, greater than or equal to, less than, less than or equal to, or equal to the threshold). The threshold may be a hard-coded value, a value set by a user, or a value that dynamically changes. In some examples, activity recognition module 118 may store or otherwise use different thresholds for different activities. In any case, activity recognition module 118 may determine whether a probability for the threshold activity satisfies a threshold. In some examples, if activity recognition module 118 determines that a probability for the physical activity satisfies a threshold, activity recognition module 118 may determine that it is likely that the user is engaged in the particular physical activity and may therefore recognize the particular physical activity as the physical activity that corresponds to the sensor data.

In some examples, activity recognition module 118 may, in response to recognizing the physical activity, cause computing device 110 to perform one or more operations associated with the physical activity, such as collecting specific data from specific sensors for the associated activity. In some examples, activity recognition module 118 may collect data from the set of sensors and store the data as physical activity information associated with the recognized physical activity (e.g., in physical activity information datastore 228 as shown in FIG. 2). Physical activity information may include data descriptive of a particular physical activity. Examples of such data include but are not limited to: time of physical activity, geographic locations at which user performs the physical activity, heartrate, number of steps taken by a user, speed or rate of change at which a user is moving, temperature of the user or the environment of the user, altitude or elevation at which the user performs the activity, to name only a few examples of physical activity information.

In some examples, activity recognition module 118 may output the physical activity information associated with the recognized physical activity to the user in a graphical user interface. In some examples, activity recognition module 118 may perform analytics on the physical activity information such as determining various statistical metrics including aggregate values, average values, and the like. In some examples, activity recognition module 118 may send the physical activity information to a remote server that associates the physical activity information with a user account of the user. In still other examples, activity recognition module 118 may notify one or more third-party applications. For instance, a third-party fitness application may register with activity recognition module 118, and the third-party fitness application can record physical activity information for a user.

FIG. 2 is a block diagram illustrating further details of a computing device 210 that performs on-device activity recognition, in accordance with one or more aspects of the present disclosure. Computing device 210 of FIG. 2 is described below as an example of computing device 110 illustrated in FIG. 1. FIG. 2 illustrates only one particular example of computing device 210, and many other examples of computing device 210 may be used in other instances and may include a subset of the components included in example computing device 210 or may include additional components not shown in FIG. 2.

As shown in the example of FIG. 2, computing device 210 includes presence-sensitive display 212, one or more processors 240, one or more input components 242, one or more communication units 244, one or more output components 246, and one or more storage components 248. Presence-sensitive display (PSD) 212 includes display component 202 and presence-sensitive input component 204. Input components 242 include sensor components 214. Storage components 248 of computing device 210 also include activity recognition module 218, activity detection model 220, application modules 224, sensor processing modules 226, and physical activity information datastore 228.

Communication channels 250 may interconnect each of the components 240, 212, 202, 204, 244, 246, 242, 214, 248, 218, 220, 224, 226, and 228 for inter-component communications (physically, communicatively, and/or operatively). In some examples, communication channels 250 may include a system bus, a network connection, an interprocess communication data structure, or any other method for communicating data.

One or more input components 242 of computing device 210 may receive input. Examples of input are tactile, audio, and video input. Input components 242 of computing device 210, in one example, includes a presence-sensitive display, touch-sensitive screen, mouse, keyboard, voice responsive system, video camera, microphone or any other type of device for detecting input from a human or machine.

One or more input components 242 include one or more sensor components 214. Numerous examples of sensor components 214 exist and include any input component configured to obtain environmental information about the circumstances surrounding computing device 210 and/or physiological information that defines the activity state and/or physical well-being of a user of computing device 210. In some examples, a sensor component may be an input component that obtains physical position, movement, and/or location information of computing device 210. For instance, sensor components 214 may include one or more location sensors 214A (GPS components, Wi-Fi components, cellular components), one or more temperature sensors 214B, one or more motion sensors 214C (e.g., multi-axial accelerometers, gyros), one or more pressure sensors 214D (e.g., barometer), one or more ambient light sensors 214E, and one or more other sensors 214F (e.g., microphone, camera, infrared proximity sensor, hygrometer, and the like). Other sensors may include a heart rate sensor, magnetometer, glucose sensor, hygrometer sensor, olfactory sensor, compass sensor, step counter sensor, to name a few other non-limiting examples.

One or more output components 246 of computing device 210 may generate output. Examples of output are tactile, audio, and video output. Output components 246 of computing device 210, in one example, includes a presence-sensitive display, sound card, video graphics adapter card, speaker, cathode ray tube (CRT) monitor, liquid crystal display (LCD), or any other type of device for generating output to a human or machine.

One or more communication units 244 of computing device 210 may communicate with external devices via one or more wired and/or wireless networks by transmitting and/or receiving network signals on the one or more networks. Examples of communication units 244 include a network interface card (e.g. such as an Ethernet card), an optical transceiver, a radio frequency transceiver, a GPS receiver, or any other type of device that can send and/or receive information. Other examples of communication units 244 may include short wave radios, cellular data radios, wireless network radios, as well as universal serial bus (USB) controllers.

Presence-sensitive display (PSD) 212 of computing device 200 includes display component 202 and presence-sensitive input component 204. Display component 202 may be a screen at which information is displayed by PSD 212 and presence-sensitive input component 204 may detect an object at and/or near display component 202. As one example range, presence-sensitive input component 204 may detect an object, such as a finger or stylus that is within two inches or less of display component 202. Presence-sensitive input component 204 may determine a location (e.g., an (x,y) coordinate) of display component 202 at which the object was detected. In another example range, presence-sensitive input component 204 may detect an object six inches or less from display component 202 and other ranges are also possible. Presence-sensitive input component 204 may determine the location of display component 202 selected by a user's finger using capacitive, inductive, and/or optical recognition techniques. In some examples, presence-sensitive input component 204 also provides output to a user using tactile, audio, or video stimuli as described with respect to display component 202. In the example of FIG. 2, PSD 212 presents a user interface.

While illustrated as an internal component of computing device 210, presence-sensitive display 212 may also represent an external component that shares a data path with computing device 210 for transmitting and/or receiving input and output. For instance, in one example, PSD 212 represents a built-in component of computing device 210 located within and physically connected to the external packaging of computing device 210 (e.g., a screen on a mobile phone). In another example, PSD 212 represents an external component of computing device 210 located outside and physically separated from the packaging of computing device 210 (e.g., a monitor, a projector, etc. that shares a wired and/or wireless data path with a tablet computer).

PSD 212 of computing device 210 may receive tactile input from a user of computing device 110. PSD 212 may receive indications of the tactile input by detecting one or more gestures from a user of computing device 210 (e.g., the user touching or pointing to one or more locations of PSD 212 with a finger or a stylus pen). PSD 212 may present output to a user. PSD 212 may present the output as a graphical user interface, which may be associated with functionality provided by computing device 210. For example, PSD 212 may present various user interfaces of components of a computing platform, operating system, applications, or services executing at or accessible by computing device 210 (e.g., an electronic message application, a navigation application, an Internet browser application, a mobile operating system, etc.). A user may interact with a respective user interface to cause computing devices 210 to perform operations relating to a function.

PSD 212 of computing device 210 may detect two-dimensional and/or three-dimensional gestures as input from a user of computing device 210. For instance, a sensor of PSD 212 may detect a user's movement (e.g., moving a hand, an arm, a pen, a stylus, etc.) within a threshold distance of the sensor of PSD 212. PSD 212 may determine a two- or three-dimensional vector representation of the movement and correlate the vector representation to a gesture input (e.g., a hand-wave, a pinch, a clap, a pen stroke, etc.) that has multiple dimensions. In other words, PSD 212 can detect a multi-dimensional gesture without requiring the user to gesture at or near a screen or surface at which PSD 212 outputs information for display. Instead, PSD 212 can detect a multi-dimensional gesture performed at or near a sensor which may or may not be located near the screen or surface at which PSD 212 outputs information for display.

One or more processors 240 may implement functionality and/or execute instructions within computing device 210. For example, processors 240 on computing device 210 may receive and execute instructions stored by storage components 248 that execute the functionality of modules 218, 224, 226, and/or 228 and model 220. The instructions executed by processors 240 may cause computing device 210 to store information within storage components 248 during program execution. Examples of processors 240 include application processors, display controllers, sensor hubs, and any other hardware configured to function as a processing unit. Processors 240 may execute instructions of modules 218, 224, 226, and/or 228 and model 220 to cause PSD 212 to render portions of content of display data as one of user interface screen shots at PSD 212. That is, modules 218, 224, 226, and/or 228 and model 220 may be operable by processors 240 to perform various actions or functions of computing device 210.

One or more storage components 248 within computing device 210 may store information for processing during operation of computing device 210 (e.g., computing device 210 may store data accessed by modules 218, 224, 226, and/or 228 and model 220 during execution at computing device 210). In some examples, storage component 248 is a temporary memory, meaning that a primary purpose of storage component 248 is not long-term storage. Storage components 248 on computing device 210 may be configured for short-term storage of information as volatile memory and therefore not retain stored contents if powered off. Examples of volatile memories include random access memories (RAM), dynamic random access memories (DRAM), static random access memories (SRAM), and other forms of volatile memories known in the art.

Storage components 248, in some examples, also include one or more computer-readable storage media. Storage components 248 may be configured to store larger amounts of information than volatile memory. Storage components 248 may further be configured for long-term storage of information as non-volatile memory space and retain information after power on/off cycles. Examples of non-volatile memories include magnetic hard discs, optical discs, floppy discs, flash memories, or forms of electrically programmable memories (EPROM) or electrically erasable and programmable (EEPROM) memories. Storage components 248 may store program instructions and/or information (e.g., data) associated with modules, 218, 224, 226, and/or 228, model 220, as well as data stores 280.

Application modules 224 represent all the various individual applications and services executing at computing device 210. A user of computing device 210 may interact with an interface (e.g., a graphical user interface) associated with one or more application modules 224 to cause computing device 210 to perform a function. Numerous examples of application modules 224 may exist and include, a fitness application, a calendar application, a personal assistant or prediction engine, a search application, a map or navigation application, a transportation service application (e.g., a bus or train tracking application), a social media application, a game application, an e-mail application, a messaging application, an Internet browser application, or any and all other applications that may execute at computing device 210. Although shown separately from application modules 224, activity recognition module 218 may be included within one or more of application modules 224 (e.g., included within a fitness application).

As shown in FIG. 2, computing device 210 may include sensor processing modules 226. In some examples, sensor processing modules 226 may receive outputs from sensor components 214 and generate sensor data that represents the outputs. For instance, each of sensor components 214 may have a corresponding sensor processing module. As an example, a sensor processing module for location sensor component 214A may generate GPS coordinate values in sensor data (e.g., objects), where the GPS coordinates are based on hardware outputs of location sensor component 214A. As another example, a sensor processing module for motion sensor component 214C may generate motion and/or acceleration values along different axes of a coordinate system in the motion data, where the motion and/or acceleration values are based on hardware outputs of motion sensor component 214C.

In FIG. 2, activity recognition module 218 may receive motion data produced by one or more motion sensors. For example, activity recognition module 218 may receive motion data produced by motion sensor 214C, which may be sensor data generated by motion sensor 214C or motion data generated by sensor processing modules 226 based on sensor data generated by motion sensor 214C. In some examples, activity recognition module 218 may receive motion data produced by one or more motion sensors of a wearable computing device communicably coupled to computing device 210, such as a wearable computing device similar to wearable computing device 100 shown in FIG. 1.

The motion data produced by one or more motion sensors, such as motion sensor 214C or the one or more motion sensors of a wearable computing device and received by activity recognition module 218 may be in the form of multi-axial accelerometer data, such as tri-axial accelerometer data. For example, the tri-axial accelerometer data may be in the form of three channels of floating point numbers that specify the acceleration measured by the one or more motion sensors along the x, y, and z axes.

The motion data produced by one or more motion sensors and received by activity recognition module 218 may be motion data sensed by the one or more motion sensors over a period of time. For example, the motion data may be motion data sensed by the one or more sensors over a period of approximately 10 seconds. Given a sampling rate of 25 hertz, the motion data may include a total of 256 samples of multi-axial accelerometer data.

Activity recognition module 218 may, in response to receiving the motion data produced by the one or more sensors, recognize, using one or more neural networks trained with differential privacy, a physical activity that corresponds to the motion data produced by the one or more motion sensors. To that end, activity recognition module 218 may use activity recognition model 220 to recognize a physical activity that corresponds to the motion data produced by the one or more motion sensors.

Activity recognition module 218 may include activity recognition model 220 which may include one or more neural networks trained off-device with differential privacy to recognize a physical activity that corresponds to the motion data produced by the one or more motion sensors. Examples of activity recognition model 220 may include one or more convolutional neural networks, recurrent neural networks, or any other suitable artificial neural network trained with differential privacy. Activity recognition module 218 may take as input the motion data produced by the one or more motion sensors, such as multi-axial accelerometer data sensed by the one or more sensors over a period of time, and may output one or more probabilities, such as in the form of a probability distribution, that correspond to one or more physical activities. That is, activity recognition model 220 may, for motion data over a period of time, generate respective probabilities for respective physical activities, such as a probability distribution of the motion data over a plurality of physical activities.

In some examples, activity recognition module 218 and/or activity recognition model 220 may, in addition to using motion data produced by the one or more motion sensors to recognize the physical activity that corresponds to the motion data produced by the one or more motion sensors, use additional sensor data produced by one or more sensor components 2214 to recognize the physical activity that corresponds to the motion data produced by the one or more motion sensors. For example, activity recognition module 218 may use heartrate data produced by a heartrate sensor that measures the heartrate of the user to augment the use of motion data produced by the one or more motion sensors to recognize the physical activity that corresponds to the motion data produced by the one or more motion sensors.

For instance, activity recognition module 218 may, for motion data produced by the one or more motion sensors for a given time period, also receive corresponding heartrate data produced by the heartrate sensor during the same given time period, and may adjust, based on the corresponding heartrate data produced by the heartrate sensor during the same given time period, the one or more probabilities that correspond to one or more physical activities as determined by activity recognition model 220. For example, if activity recognition module 218 determines that the heartrate of the user during the given time period is within a specified range of the resting heartrate of the user, activity recognition module 218 may increase the probability of the user being still and may decrease the probability of the user being active, such as by decreasing the probabilities of the user walking, cycling, or running. In another example, if activity recognition module 218 determines that the heartrate of the user during the given time period is outside of a specified range of the resting heartrate of the user, activity recognition module 218 may increase the probability of the user being active, such as by increasing the probabilities of the user walking, cycling, or running, and may decrease the probability that the user is being still.

Activity recognition module 218 may recognize, based at least in part on the determined one or more probabilities that correspond to one or more physical activities, the physical activity that corresponds to the motion data produced by the one or more motion sensors. In some examples, activity recognition module 218 may determine that the physical activity associated with the highest probability out of the one or more probabilities that correspond to one or more physical activities as determined by activity recognition model 220, is the physical activity that corresponds to the motion data produced by the one or more motion sensors. For example, if activity recognition model 220 determines a probability of 0.6 that the physical activity is walking, a probability of 0.2 that the physical activity is running, a probability of 0.2 that the physical activity is riding a bicycle, and a probability of 0.0 that the physical activity is remaining still (i.e., not moving), activity recognition module 218 may recognize the physical activity that corresponds to the motion data is walking.

In some examples, activity recognition module 218 may determine that the physical activity associated with the highest probability out of the one or more probabilities that correspond to one or more physical activities as determined by activity recognition model 220, is the physical activity that corresponds to the motion data produced by the one or more motion sensors if the associated probability of the physical activity satisfies a threshold (e.g., a probability is greater than, greater than or equal to, less than, less than or equal to, or equal to the threshold). If activity detection module 218 determines that a probability for the physical activity satisfies a threshold, activity recognition module 218 may determine that it is likely that the user is engaged in the particular physical activity and may therefore recognize the particular physical activity as the physical activity that corresponds to the motion data.

Activity recognition module 218 may, in response to recognizing the physical activity that corresponds to the motion data, perform one or more operations associated with the physical activity. In some examples, activity recognition module 218 may, in response to recognizing the physical activity that corresponds to the motion data, perform fitness tracking of the user, such as by tracking physical activity information associated with the physical activity, such as the time of physical activity, geographic locations at which user performs the physical activity, heartrate, number of steps taken by a user, speed or rate of change at which a user is moving, temperature of the user or the environment of the user, altitude or elevation at which the user performs the activity, and the like

In some examples, activity recognition module 218 may store an indication of the physical activity in storage components 248, such as in physical activity information datastore 228, along with physical activity information associated with the physical activity, such as the time of physical activity, geographic locations at which user performs the physical activity, heartrate, number of steps taken by a user, speed or rate of change at which a user is moving, temperature of the user or the environment of the user, altitude or elevation at which the user performs the activity, and the like.

In some examples, activity recognition module 218 may perform analytics on the physical activity information associated with the physical activity, such as determining various statistical metrics including aggregate values, average values, and the like. In some examples, activity recognition module 218 may send the physical activity information associated with the physical activity to a remote server that associates the physical activity information with a user account of the user. In still other examples, activity recognition module 218 may notify one or more third-party applications. For instance, a third-party fitness application may register with activity recognition module 218, and the third-party fitness application can record physical activity information for a user.

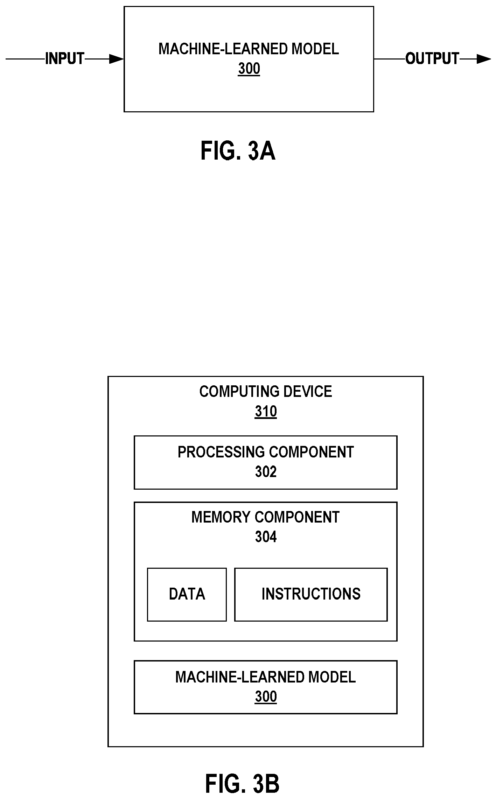

FIGS. 3A-3E are conceptual diagrams illustrating aspects of an example machine-learned model according to example implementations of the present disclosure. FIGS. 3A-3E are described below in the context of activity recognition module 218 of FIG. 2. For example, in some instances, machine-learned model 300, as referenced below, may be an example of activity recognition model 220 of FIG. 2.

FIG. 3A depicts a conceptual diagram of an example machine-learned model according to example implementations of the present disclosure. As illustrated in FIG. 3A, in some implementations, machine-learned model 300 is trained to receive input data of one or more types and, in response, provide output data of one or more types. Thus, FIG. 3A illustrates machine-learned model 300 performing inference. For example, the input data received by machine-learned model 300 may be motion data, such as sensor data generated by multi-axial accelerometers, and the output data provided by machine-learned model 300 may be an activity of a user that corresponds to the motion data.

The input data may include one or more features that are associated with an instance or an example. In some implementations, the one or more features associated with the instance or example can be organized into a feature vector. In some implementations, the output data can include one or more predictions. Predictions can also be referred to as inferences. Thus, given features associated with a particular instance, machine-learned model 300 can output a prediction for such instance based on the features.

Machine-learned model 300 can be or include one or more of various different types of machine-learned models. In particular, in some implementations, machine-learned model 300 can perform classification, regression, clustering, anomaly detection, recommendation generation, and/or other tasks.

In some implementations, machine-learned model 300 can perform various types of classification based on the input data. For example, machine-learned model 300 can perform binary classification or multiclass classification. In binary classification, the output data can include a classification of the input data into one of two different classes. In multiclass classification, the output data can include a classification of the input data into one (or more) of more than two classes. The classifications can be single label or multi-label. Machine-learned model 300 may perform discrete categorical classification in which the input data is simply classified into one or more classes or categories.

In some implementations, machine-learned model 300 can perform classification in which machine-learned model 300 provides, for each of one or more classes, a numerical value descriptive of a degree to which it is believed that the input data should be classified into the corresponding class. In some instances, the numerical values provided by machine-learned model 300 can be referred to as "confidence scores" that are indicative of a respective confidence associated with classification of the input into the respective class. In some implementations, the confidence scores can be compared to one or more thresholds to render a discrete categorical prediction. In some implementations, only a certain number of classes (e.g., one) with the relatively largest confidence scores can be selected to render a discrete categorical prediction.

Machine-learned model 300 may output a probabilistic classification. For example, machine-learned model 300 may predict, given a sample input, a probability distribution over a set of classes. Thus, rather than outputting only the most likely class to which the sample input should belong, machine-learned model 300 can output, for each class, a probability that the sample input belongs to such class. In some implementations, the probability distribution over all possible classes can sum to one. In some implementations, a Softmax function, or other type of function or layer can be used to squash a set of real values respectively associated with the possible classes to a set of real values in the range (0, 1) that sum to one.

In some examples, the probabilities provided by the probability distribution can be compared to one or more thresholds to render a discrete categorical prediction. In some implementations, only a certain number of classes (e.g., one) with the relatively largest predicted probability can be selected to render a discrete categorical prediction.

In cases in which machine-learned model 300 performs classification, machine-learned model 300 may be trained using supervised learning techniques. For example, machine-learned model 300 may be trained on a training dataset that includes training examples labeled as belonging (or not belonging) to one or more classes. Further details regarding supervised training techniques are provided below in the descriptions of FIGS. 3B through 3E.

In some implementations, machine-learned model 300 can perform regression to provide output data in the form of a continuous numeric value. The continuous numeric value can correspond to any number of different metrics or numeric representations, including, for example, currency values, scores, or other numeric representations. As examples, machine-learned model 300 can perform linear regression, polynomial regression, or nonlinear regression. As examples, machine-learned model 300 can perform simple regression or multiple regression. As described above, in some implementations, a Softmax function or other function or layer can be used to squash a set of real values respectively associated with two or more possible classes to a set of real values in the range (0, 1) that sum to one.

Machine-learned model 300 may, in some cases, act as an agent within an environment. For example, machine-learned model 300 can be trained using reinforcement learning, which will be discussed in further detail below.

In some implementations, machine-learned model 300 can be a parametric model while, in other implementations, machine-learned model 300 can be a non-parametric model. In some implementations, machine-learned model 300 can be a linear model while, in other implementations, machine-learned model 300 can be a non-linear model.

As described above, machine-learned model 300 can be or include one or more of various different types of machine-learned models. Examples of such different types of machine-learned models are provided below for illustration. One or more of the example models described below can be used (e.g., combined) to provide the output data in response to the input data. Additional models beyond the example models provided below can be used as well.

In some implementations, machine-learned model 300 can be or include one or more classifier models such as, for example, linear classification models; quadratic classification models; etc. Machine-learned model 300 may be or include one or more regression models such as, for example, simple linear regression models; multiple linear regression models; logistic regression models; stepwise regression models; multivariate adaptive regression splines; locally estimated scatterplot smoothing models; etc.

In some implementations, machine-learned model 300 can be or include one or more artificial neural networks (also referred to simply as neural networks). A neural network can include a group of connected nodes, which also can be referred to as neurons or perceptrons. A neural network can be organized into one or more layers. Neural networks that include multiple layers can be referred to as "deep" networks. A deep network can include an input layer, an output layer, and one or more hidden layers positioned between the input layer and the output layer. The nodes of the neural network can be connected or non-fully connected.

Machine-learned model 300 can be or include one or more feed forward neural networks. In feed forward networks, the connections between nodes do not form a cycle. For example, each connection can connect a node from an earlier layer to a node from a later layer.

In some instances, machine-learned model 300 can be or include one or more recurrent neural networks. In some instances, at least some of the nodes of a recurrent neural network can form a cycle. Recurrent neural networks can be especially useful for processing input data that is sequential in nature. In particular, in some instances, a recurrent neural network can pass or retain information from a previous portion of the input data sequence to a subsequent portion of the input data sequence through the use of recurrent or directed cyclical node connections.

In some examples, sequential input data can include time-series data (e.g., sensor data versus time or imagery captured at different times). For example, a recurrent neural network can analyze sensor data versus time to detect or predict a swipe direction, to perform handwriting recognition, etc. Sequential input data may include words in a sentence (e.g., for natural language processing, speech detection or processing, etc.); notes in a musical composition; sequential actions taken by a user (e.g., to detect or predict sequential application usage); sequential object states; etc.

Example recurrent neural networks include long short-term (LSTM) recurrent neural networks; gated recurrent units; bi-direction recurrent neural networks; continuous time recurrent neural networks; neural history compressors; echo state networks; Elman networks; Jordan networks; recursive neural networks; Hopfield networks; fully recurrent networks; sequence-to- sequence configurations; etc.

In some implementations, machine-learned model 300 can be or include one or more convolutional neural networks. In some instances, a convolutional neural network can include one or more convolutional layers that perform convolutions over input data using learned filters.

Filters can also be referred to as kernels. Convolutional neural networks can be especially useful for vision problems such as when the input data includes imagery such as still images or video. However, convolutional neural networks can also be applied for natural language processing.

In some examples, machine-learned model 300 can be or include one or more generative networks such as, for example, generative adversarial networks. Generative networks can be used to generate new data such as new images or other content.

Machine-learned model 300 may be or include an autoencoder. In some instances, the aim of an autoencoder is to learn a representation (e.g., a lower- dimensional encoding) for a set of data, typically for the purpose of dimensionality reduction. For example, in some instances, an autoencoder can seek to encode the input data and then provide output data that reconstructs the input data from the encoding. Recently, the autoencoder concept has become more widely used for learning generative models of data. In some instances, the autoencoder can include additional losses beyond reconstructing the input data.

Machine-learned model 300 may be or include one or more other forms of artificial neural networks such as, for example, deep Boltzmann machines; deep belief networks; stacked autoencoders; etc. Any of the neural networks described herein can be combined (e.g., stacked) to form more complex networks.

One or more neural networks can be used to provide an embedding based on the input data. For example, the embedding can be a representation of knowledge abstracted from the input data into one or more learned dimensions. In some instances, embeddings can be a useful source for identifying related entities. In some instances, embeddings can be extracted from the output of the network, while in other instances embeddings can be extracted from any hidden node or layer of the network (e.g., a close to final but not final layer of the network). Embeddings can be useful for performing auto suggest next video, product suggestion, entity or object recognition, etc. In some instances, embeddings are useful inputs for downstream models. For example, embeddings can be useful to generalize input data (e.g., search queries) for a downstream model or processing system.

In some implementations, machine-learned model 300 can perform one or more dimensionality reduction techniques such as, for example, principal component analysis; kernel principal component analysis; graph-based kernel principal component analysis; principal component regression; partial least squares regression; Sammon mapping; multidimensional scaling; projection pursuit; linear discriminant analysis; mixture discriminant analysis; quadratic discriminant analysis; generalized discriminant analysis; flexible discriminant analysis; autoencoding; etc.

In some implementations, machine-learned model 300 can perform or be subjected to one or more reinforcement learning techniques such as Markov decision processes; dynamic programming; Q functions or Q-learning; value function approaches; deep Q-networks; differentiable neural computers; asynchronous advantage actor-critics; deterministic policy gradient; etc.

In some implementations, machine-learned model 300 can be an autoregressive model. In some instances, an autoregressive model can specify that the output data depends linearly on its own previous values and on a stochastic term. In some instances, an autoregressive model can take the form of a stochastic difference equation. One example autoregressive model is WaveNet, which is a generative model for raw audio.

In some implementations, machine-learned model 300 can include or form part of a multiple model ensemble. As one example, bootstrap aggregating can be performed, which can also be referred to as "bagging." In bootstrap aggregating, a training dataset is split into a number of subsets (e.g., through random sampling with replacement) and a plurality of models are respectively trained on the number of subsets. At inference time, respective outputs of the plurality of models can be combined (e.g., through averaging, voting, or other techniques) and used as the output of the ensemble.

One example ensemble is a random forest, which can also be referred to as a random decision forest. Random forests are an ensemble learning method for classification, regression, and other tasks. Random forests are generated by producing a plurality of decision trees at training time. In some instances, at inference time, the class that is the mode of the classes (classification) or the mean prediction (regression) of the individual trees can be used as the output of the forest. Random decision forests can correct for decision trees' tendency to overfit their training set.

Another example ensemble technique is stacking, which can, in some instances, be referred to as stacked generalization. Stacking includes training a combiner model to blend or otherwise combine the predictions of several other machine-learned models. Thus, a plurality of machine-learned models (e.g., of same or different type) can be trained based on training data. In addition, a combiner model can be trained to take the predictions from the other machine-learned models as inputs and, in response, produce a final inference or prediction. In some instances, a single-layer logistic regression model can be used as the combiner model.

Another example ensemble technique is boosting. Boosting can include incrementally building an ensemble by iteratively training weak models and then adding to a final strong model. For example, in some instances, each new model can be trained to emphasize the training examples that previous models misinterpreted (e.g., misclassified). For example, a weight associated with each of such misinterpreted examples can be increased. One common implementation of boosting is AdaBoost, which can also be referred to as Adaptive Boosting. Other example boosting techniques include LPBoost; TotalBoost; BrownBoost; xgboost; MadaBoost, LogitBoost, gradient boosting; etc. Furthermore, any of the models described above (e.g., regression models and artificial neural networks) can be combined to form an ensemble. As an example, an ensemble can include a top level machine-learned model or a heuristic function to combine and/or weight the outputs of the models that form the ensemble.

In some implementations, multiple machine-learned models (e.g., that form an ensemble can be linked and trained jointly (e.g., through backpropagation of errors sequentially through the model ensemble). However, in some implementations, only a subset (e.g., one) of the jointly trained models is used for inference.

In some implementations, machine-learned model 300 can be used to preprocess the input data for subsequent input into another model. For example, machine-learned model 300 can perform dimensionality reduction techniques and embeddings (e.g., matrix factorization, principal components analysis, singular value decomposition, word2vec/GLOVE, and/or related approaches); clustering; and even classification and regression for downstream consumption. Many of these techniques have been discussed above and will be further discussed below.

As discussed above, machine-learned model 300 can be trained or otherwise configured to receive the input data and, in response, provide the output data. The input data can include different types, forms, or variations of input data. As examples, in various implementations, the input data can include features that describe the content (or portion of content) initially selected by the user, e.g., content of user-selected document or image, links pointing to the user selection, links within the user selection relating to other files available on device or cloud, metadata of user selection, etc. Additionally, with user permission, the input data includes the context of user usage, either obtained from the app itself or from other sources. Examples of usage context include breadth of share (sharing publicly, or with a large group, or privately, or a specific person), context of share, etc. When permitted by the user, additional input data can include the state of the device, e.g., the location of the device, the apps running on the device, etc.

Additionally, with user permission, the input data includes the context of user usage, either obtained from the app itself or from other sources. Examples of usage context include breadth of share (sharing publicly, or with a large group, or privately, or a specific person), context of share, etc. When permitted by the user, additional input data can include the state of the device, e.g., the location of the device, the apps running on the device, etc.

In some implementations, machine-learned model 300 can receive and use the input data in its raw form. In some implementations, the raw input data can be preprocessed. Thus, in addition or alternatively to the raw input data, machine-learned model 300 can receive and use the preprocessed input data.

In some implementations, preprocessing the input data can include extracting one or more additional features from the raw input data. For example, feature extraction techniques can be applied to the input data to generate one or more new, additional features. Example feature extraction techniques include edge detection; corner detection; blob detection; ridge detection; scale-invariant feature transform; motion detection; optical flow; Hough transform; etc.

In some implementations, the extracted features can include or be derived from transformations of the input data into other domains and/or dimensions. As an example, the extracted features can include or be derived from transformations of the input data into the frequency domain. For example, wavelet transformations and/or fast Fourier transforms can be performed on the input data to generate additional features.

In some implementations, the extracted features can include statistics calculated from the input data or certain portions or dimensions of the input data. Example statistics include the mode, mean, maximum, minimum, or other metrics of the input data or portions thereof.

In some implementations, as described above, the input data can be sequential in nature. In some instances, the sequential input data can be generated by sampling or otherwise segmenting a stream of input data. As one example, frames can be extracted from a video. In some implementations, sequential data can be made non-sequential through summarization.

As another example preprocessing technique, portions of the input data can be imputed. For example, additional synthetic input data can be generated through interpolation and/or extrapolation.

As another example preprocessing technique, some or all of the input data can be scaled, standardized, normalized, generalized, and/or regularized. Example regularization techniques include ridge regression; least absolute shrinkage and selection operator (LASSO); elastic net; least-angle regression; cross-validation; L1 regularization; L2 regularization; etc. As one example, some or all of the input data can be normalized by subtracting the mean across a given dimension's feature values from each individual feature value and then dividing by the standard deviation or other metric.

As another example preprocessing technique, some or all or the input data can be quantized or discretized. In some cases, qualitative features or variables included in the input data can be converted to quantitative features or variables. For example, one hot encoding can be performed.

In some examples, dimensionality reduction techniques can be applied to the input data prior to input into machine-learned model 300. Several examples of dimensionality reduction techniques are provided above, including, for example, principal component analysis; kernel principal component analysis; graph-based kernel principal component analysis; principal component regression; partial least squares regression; Sammon mapping; multidimensional scaling; projection pursuit; linear discriminant analysis; mixture discriminant analysis; quadratic discriminant analysis; generalized discriminant analysis; flexible discriminant analysis; autoencoding; etc.

In some implementations, during training, the input data can be intentionally deformed in any number of ways to increase model robustness, generalization, or other qualities. Example techniques to deform the input data include adding noise; changing color, shade, or hue; magnification; segmentation; amplification; etc.

In response to receipt of the input data, machine-learned model 300 can provide the output data. The output data can include different types, forms, or variations of output data. As examples, in various implementations, the output data can include content, either stored locally on the user device or in the cloud, that is relevantly shareable along with the initial content selection.

As discussed above, in some implementations, the output data can include various types of classification data (e.g., binary classification, multiclass classification, single label, multi- label, discrete classification, regressive classification, probabilistic classification, etc.) or can include various types of regressive data (e.g., linear regression, polynomial regression, nonlinear regression, simple regression, multiple regression, etc.). In other instances, the output data can include clustering data, anomaly detection data, recommendation data, or any of the other forms of output data discussed above.

In some implementations, the output data can influence downstream processes or decision making. As one example, in some implementations, the output data can be interpreted and/or acted upon by a rules-based regulator.

The present disclosure provides systems and methods that include or otherwise leverage one or more machine-learned models to suggest content, either stored locally on the user's device or in the cloud, that is relevantly shareable along with the initial content selection based on features of the initial content selection. Any of the different types or forms of input data described above can be combined with any of the different types or forms of machine-learned models described above to provide any of the different types or forms of output data described above.

The systems and methods of the present disclosure can be implemented by or otherwise executed on one or more computing devices. Example computing devices include user computing devices (e.g., laptops, desktops, and mobile computing devices such as tablets, smartphones, wearable computing devices, etc.); embedded computing devices (e.g., devices embedded within a vehicle, camera, image sensor, industrial machine, satellite, gaming console or controller, or home appliance such as a refrigerator, thermostat, energy meter, home energy manager, smart home assistant, etc.); other computing devices; or combinations thereof.

FIG. 3B illustrates a conceptual diagram of computing device 310, which is an example of computing device 110 of FIG. 1. Computing device 310 includes processing component 302, memory component 304 and machine-learned model 300. Computing device 310 may store and implement machine-learned model 300 locally (i.e., on-device). Thus, machine-learned model 300 can be stored at and/or implemented locally by an embedded device or a user computing device such as a mobile device. Output data obtained through local implementation of machine-learned model 300 at the embedded device or the user computing device can be used to improve performance of the embedded device or the user computing device (e.g., an application implemented by the embedded device or the user computing device).

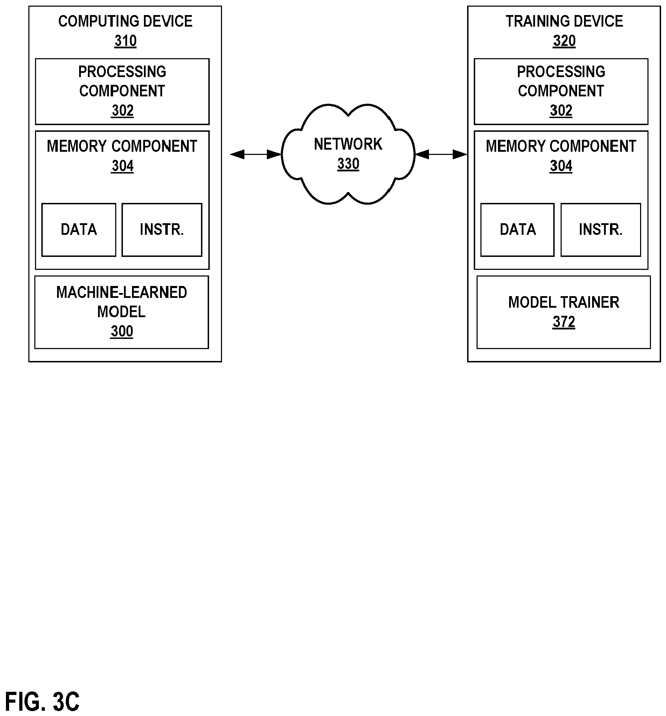

FIG. 3C illustrates a conceptual diagram of an example computing device in communication with an example training computing system that includes a model trainer. FIG. 3C includes client device 310 communicating with training device 320 over network 330. Client device 310 is an example of computing device 110 of FIG. 1. Machine-learned model 300 described herein can be trained at a training computing system, such as training device 320, and then provided for storage and/or implementation at one or more computing devices, such as client device 310. For example, model trainer 372 executes locally at training device 320. In some examples, training device 320, including model trainer 372, can be included in or separate from client device 310 or any other computing device that implements machine-learned model 300.

Computing device 310 that implements machine-learned model 300 or other aspects of the present disclosure and training device 320 that trains machine-learned model 300 can include a number of hardware components that enable performance of the techniques described herein. For example, computing device 310 can include one or more memory devices that store some or all of machine-learned model 300. For example, machine-learned model 300 can be a structured numerical representation that is stored in memory. The one or more memory devices can also include instructions for implementing machine-learned model 300 or performing other operations. Example memory devices include RAM, ROM, EEPROM, EPROM, flash memory devices, magnetic disks, etc., and combinations thereof.

Computing device 310 can also include one or more processing devices that implement some or all of machine-learned model 300 and/or perform other related operations. Example processing devices include one or more of: a central processing unit (CPU); a visual processing unit (VPU); a graphics processing unit (GPU); a tensor processing unit (TPU); a neural processing unit (NPU); a neural processing engine; a core of a CPU, VPU, GPU, TPU, NPU or other processing device; an application specific integrated circuit (ASIC); a field programmable gate array (FPGA); a co-processor; a controller; or combinations of the processing devices described above. Processing devices can be embedded within other hardware components such as, for example, an image sensor, accelerometer, etc.

Training device 320 may perform graph processing techniques or other machine learning techniques using one or more machine learning platforms, frameworks, and/or libraries, such as, for example, TensorFlow, Caffe/Caffe2, Theano, Torch/PyTorch, MXnet, CNTK, etc. In some implementations, machine-learned model 300 may be trained in an offline fashion or an online fashion. In offline training (also known as batch learning), machine-learned model 300 is trained on the entirety of a static set of training data. In online learning, machine-learned model 300 is continuously trained (or re-trained) as new training data becomes available (e.g., while the model is used to perform inference).

Model trainer 372 may perform centralized training of machine-learned model 300 (e.g., based on a centrally stored dataset). In other implementations, decentralized training techniques such as distributed training, federated learning, or the like can be used to train, update, or personalize machine-learned model 300.

Machine-learned model 300 described herein can be trained according to one or more of various different training types or techniques. For example, in some implementations, machine-learned model 300 can be trained by model trainer 372 using supervised learning, in which machine-learned model 300 is trained on a training dataset that includes instances or examples that have labels. The labels can be manually applied by experts, generated through crowd-sourcing, or provided by other techniques (e.g., by physics-based or complex mathematical models). In some implementations, if the user has provided consent, the training examples can be provided by the user computing device. In some implementations, this process can be referred to as personalizing the model.

Once training device 320 has finished training machine-learned model 300, machine-learned model 300 may be installed onto client device 310. For example, training device 320 may transfer machine-learned model 300 to client device 310 via network 330, or machine-learned model 300 may be installed in client device 310 during manufacturing of client device 310. In some examples, once machine-learned model 300 has been trained at training device 320, training device 320 may perform post-training weight quantization, such as by using TensorFlow Lite libraries, to compress model weights, such as by compressing model weights into 8-bit integers, to enable client device 310 to perform more efficient on-device inference using machine-learned model 300.

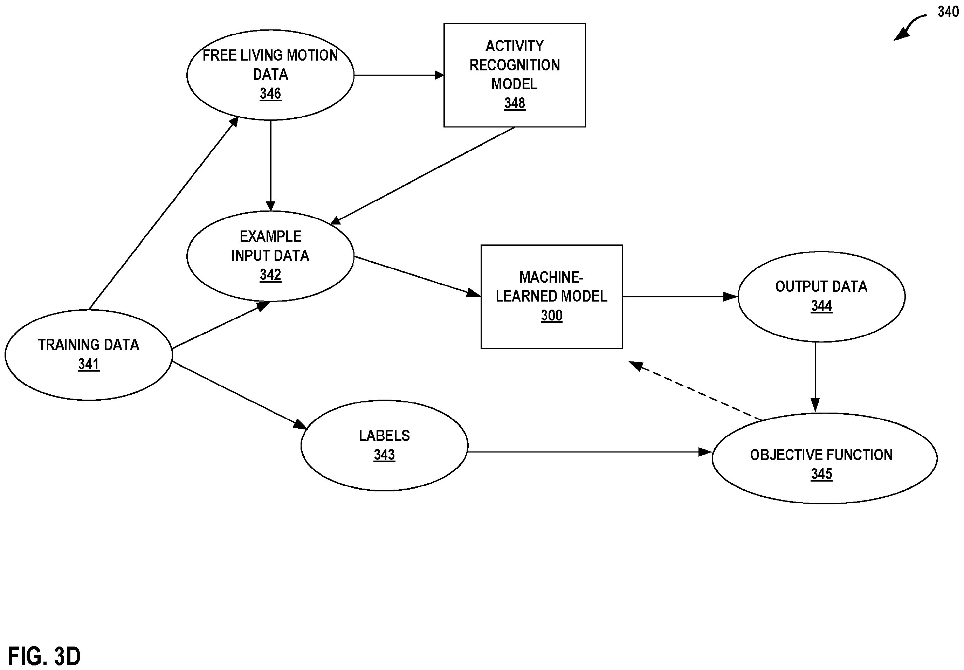

FIG. 3D illustrates a conceptual diagram of training process 340 which is an example training process in which training device 340 may train machine-learned model 300 on training data 341 that includes example input data 342 that has labels 343. Training processes 340 is one example training process; other training processes may be used as well.

Training data 341 used by training process 340 can include, upon user permission for use of such data for training, anonymized usage logs of sharing flows, e.g., content items that were shared together, bundled content pieces already identified as belonging together, e.g., from entities in a knowledge graph, etc. In some implementations, training data 341 can include examples of input data 342 that have been assigned labels 343 that correspond to output data 344.

In accordance with aspects of the present disclosure, machine-learned model 300 is trained with differential privacy. That is, machine-learned model 300 may train machine-learned model 300 in ways that offer strong mathematical guarantees that machine learned model 300 does not learn or remember the details about any specific user whose data was used (e.g., as part of example input data 342 and/or training data 341) to train machine-learned model 300, thereby preventing malicious actors from accurately determining whether a specific piece of data was used during training of machine-learned model 300, thereby preserving the privacy of users whose data were used to train machine-learned model 300.

To train machine-learned model 300 with differential privacy, training device 320 may add noise in training data 341 to hide individual examples in training data 341 of machine-learned model 300. To that end, training device 320 may train machine-learned model 300 using a differential privacy framework, such as TensorFlow Privacy. By training machine-learned model 300 using a differential privacy framework, training device 320 adds noise in training data 341 in order to provide a strong privacy guarantee that machine learned model 300 does not learn or remember the details about any specific user whose data was used (e.g., as part of input data 342 and/or training data 341) to train machine-learned model 300

A differential privacy framework used to train machine-learned model 300 using differential privacy to provide strong mathematical guarantees that machine-learned model 300 does not learn or remember the details about any specific user whose data was used to train machine-learned model 300 can be described using an epsilon (ε) parameter and a delta (δ) parameter. The value of the epsilon parameter may be a measure of the strength of the privacy guarantee, and may be a ceiling (i.e., an upper bound) on how much the probability of a particular output can increase by including or removing a single training example from training data 341. In general, the value of the epsilon parameter may be less than 10, or less than 1 for more stringent privacy guarantees. The value of the delta parameter may bound the probability of the privacy guarantee not holding. In some examples, the delta parameter may be set to a value that is the inverse of the size of training data 341, or may be set to a value that is less than the inverse of the size of training data 341.

Training device 320 may specify a target value for the delta parameter used by the differential privacy framework for training machine-learned model 300 using differential privacy. Training device 320 may determine, based on a target value for the delta parameter that is specified for machine-learned model 300 and a given set of hyperparameters, the value of the epsilon parameter used by the differential privacy framework for training machine-learned model 300 using differential privacy.

To determine the value of the epsilon parameter for machine-learned model 300, training device 320 may, in some examples, calculate the Rényi divergence (also known as Rényi entropy) of the training data for machine-learned model 300 and a neighbor of the training data. The neighbor may be a distribution of the training data that is very similar to the training data (e.g., having a Hamming distance of 1 or another analogous value). The Rényi divergence of the data may essentially be a generalized Kullback-Leibler (KL) divergence parameterized by an alpha value, where the KL divergence may be precisely derived from the Rényi divergence if the alpha value is set to 1.

If the training data has a large Rényi divergence, the distribution of the training data may shift significantly by changing a single variable. As such, it may be easier to retrieve information in this manner as a single extra example could be identifiable, thereby compromising the overall privacy. If the training data has a small Rényi divergence, an extra example may not significantly change the data, which may make it harder to distinguish whether the extra example was added to the data. As described above, the value of the delta parameter may be set to a value that is the inverse of the size of training data 341, therefore enabling the training data for machine-learned model 300 to have a relatively small Rényi divergence.

As described above, machine-learned model 300 may comprise one or more neural networks that performs activity recognition. That is, machine-learned model 300 may take, as the input, motion data that corresponds to movement and may, in response, output an indication of the physical activity that corresponds with the inputted motion data. The motion data that is inputted into machine-learned model 300 may include multi-axial accelerometer data generated by one or more motion sensors, such as one or more accelerometers. In some examples, the one or more accelerometers may generate tri-axial accelerometer data, which may be in the form of three channels of floating point numbers that specify the acceleration forces measured by the one or more accelerometers along the x, y, and z axes.

Machine-learned model 300 may be trained to perform activity recognition based on the motion data over a period of time generated by the one or more motion sensors. For example, the motion data that machine-learned model 300 may comprise approximately 10 seconds of tri-axial accelerometer data. Given a sampling rate of 25 hertz over approximately 10 seconds, machine-learned model 300 may receive a total of 256 samples of tri-axial accelerometer data, where each sample of tri-axial accelerometer data may be in the form of three channels of floating point numbers that specify the acceleration forces measured by the one or more accelerometers along the x, y, and z axes, and may be trained to determine a physical activity that corresponds to the 256 samples of tri-axial accelerometer data.

Accordingly, each individual example input data in example input data 342 may be motion data over a specified period of time, such as 10 seconds of tri-axial accelerometer data comprising a total of 256 samples of tri-axial accelerometer data. Example input data 342 may include millions of individual example input data, and the delta value for machine-learned model 300 described above may be the inverse of the number of individual example input data in input data 342.

As described above, training data 341 includes example input data 342 that has labels 343, so that each individual example input data of example input data 342 has a label of labels 343. To train machine-learned model 300 to perform activity recognition, each individual example input data of example input data 342 has a label that indicates the activity that corresponds to the individual example input data. For example, if machine-learned model 300 is trained to recognize motion data as one of riding a bicycle, running, walking, or being still (e.g., sitting, standing, and not moving), each example input data that corresponds to riding a bicycle may have a label indicative of riding a bicycle, each example input data that corresponds to running may have a label indicative of running, each example input data that corresponds to walking may have a label indicative of walking, and each example input data that corresponds to remaining still may have a label indicative of remaining still.

Example input data 342 may be motion data generated by motion sensors of computing devices carried by or worn by users as the users perform various physical activities that machine-learned model 300 is trained t o recognize. For example, example input data that corresponds to riding a bicycle may be motion data generated by motion sensors of wearable computing devices worn by users while the users were riding bicycles, example input data that corresponds to walking may be motion data generated by motion sensors of wearable computing devices worn by users while the users were walking, example input data that corresponds to running may be motion data generated by motion sensors of wearable computing devices worn by users while the users were running, and example input data that corresponds to remaining still may be motion data generated by motion sensors of wearable computing devices worn by users while the users were remaining still.

Training data 341 may include an approximately equal number of example input data of each of the plurality of physical activities that machine learned model 300 is trained to recognize. For example, if machine-learned model 300 is trained to recognize motion data as one of riding a bicycle, running, walking, or remaining still, approximately ¼ of example input data 342 may be example input data that corresponds to riding a bicycle, approximately ¼ of example input data 342 may be example input data that corresponds to running, approximately ¼ of example input data 342 may be example input data that corresponds to walking, and approximately ¼ of example input data 342 may be example input data that corresponds to remaining still. Training device 320 may resample training data 341 to address any imbalances in example input data 342 to ensure that training data 341 includes an approximately equal number of example input data of each of the plurality of physical activities that machine learned model 300 is trained to recognize.

In some examples, training device 320 may transform example input data 342 with unconstrained random rotation in all three directions (i.e., x, y, and z axes) to train machine learned model 300 to provide rotational invariance when performing activity recognition. That is, training device 320 may, for each example input data of example input data 342, apply a random rotation to the example input data, such as by applying a random rotation to the acceleration forces along the x, y, and z axes specified by the example input data, and may include the randomly rotated example input data as part of training data 341 used to train machine-learned model 300. Because the positions and orientations of motion sensors of computing devices may depend on how such computing devices are carried or worn by the user and may change over time, machine-learned model 300 that is trained to provide rotational invariance may be able to accurately perform activity recognition regardless of the position and orientation of motion sensors that generate the motion data used by machine-learned model 300 to perform activity recognition. In some examples, training device 320 may also perform reflection translation example input data 342, so that machine-learned model 300 may be able to accurately perform activity recognition regardless of whether the motion sensors (e.g., on a smart watch) that generate the motion data used by machine-learned model 300 to perform activity recognition is worn on the left or right wrists of users.

In some examples, the example input data that corresponds to remaining still may include motion data generated by users that were driving or sitting in vehicles, such as users driving or sitting in cars, buses, trains, and the like. Specifically, the example input data may include motion data generated by motion sensors of computing devices carried by or worn by users that were driving or sitting in vehicles that are labeled as remaining still. By using such example input data to train machine-learned model 300, machine-learned model 300 may be trained to not recognize such motion data generated by motion sensors of computing devices carried by or worn by users that were driving or sitting in vehicles as walking, running, or any form of physical exercise.

In some examples, the example input data that corresponds to walking may include motion data generated by motion sensors of smart phones carried by users that were walking as well as motion data generated by motion sensors of wearable computing devices worn by users that were walking. Including motion data generated by motion sensors of smart phones carried by users that were walking as well as motion data generated by motion sensors of wearable computing devices may increase the availability of training data for walking, as opposed to using only motion data generated by motion sensors of wearable computing devices such as smart watches.

In some examples, training device 320 may incorporate motion data from free living motion data 346 into training data 341 used to train machine-learned model 300. Free living motion data 346 may include motion data generated by motion sensors of computing devices carried by or worn by users as the users go about their everyday activities for multiple consecutive days, such as for three days or more. In general, free living motion data 346 may be unlabeled. As such, training device 320 may determine labels for free living motion data 346 and may include at least a portion of free living motion data 346 and its associated labels in training data 341 used to train machine-learned model 300.

In some examples, training device 320 may determine, based at least in part on the accelerometer magnitudes (i.e., the acceleration forces along the x, y, and z axes) and/or the average accelerometer magnitudes (i.e., the average of the total acceleration forces combining the x, y, and z axes) associated with windows of motion data in free living motion data 346, labels associated with the motion data in free living motion data 346. In particular, training device 320 may determine, based at least in part on the accelerometer magnitudes and/or the average accelerometer magnitudes of motion data in free living motion data 346, whether to label the motion data in free living motion data 346 and whether to select motion data in free living motion data 346 to include in training data 341 used to train machine-learned model 300.

Training device 320 may divide free living motion data 346 into windows of motion data. For example, each window may be approximately 5 seconds of motion data which, when sampled at 100 hertz, includes 512 samples of motion data, such as tri-axial accelerometer data. Training device 320 may determine an average (i.e., mean) accelerometer magnitude for the window, and may for each sample of motion data, subtract the average accelerometer magnitude for the window from the sample of motion data to produce an average accelerometer magnitude with zero mean in the window, such as to remove the effects of gravity. The average accelerometer magnitude of the clipped window may represent the overall score for the window.

Once training device 320 has produced a window having an average accelerometer magnitude with zero mean, training device 320 may clip the accelerometer magnitudes in the window between two values, such as between 0 and 4, to remove motion below the mean and to reduce the effect of outlier accelerometer magnitude values. Training device 320 may therefore determine the average clipped accelerometer magnitude in the window as an overall score for the window.

The overall score for a window may correspond with the level of activity detected within the window, where a low overall score for a window may correspond with a low level of activity detected within the window and a high overall score for a window may correspond with a high level of activity. For example, a window may be classified based on the overall score for the window as one of four levels of activity: being still, low intensity, medium intensity, and high intensity. Training device 320 may therefore select motion data of free living motion data 346 in windows classified as being still for inclusion in example input data of example input data 342 that corresponds to being still used to train machine-learned model 300.

In some examples, training device 320 may also select motion data of free living motion data 346 in windows classified as low intensity activity for inclusion in example input data of example input data 342 that corresponds to being still used to train machine-learned model 300. Including such motion data that corresponds to low intensity activity in example input data of example input data 342 that corresponds to remaining still used to train machine-learned model 300 may enable machine-learned model 300 to perform better as a general activity classifier, as well as to improve machine-learned model 300's stillness detection in general.

In some examples, training device 320 may perform activity recognition of free motion living data 346 using a pre-trained activity recognition model, such as activity recognition model 348, to label free motion living data 346 and to include the labeled free motion living data 346 in training data 341 used to train machine-learned model 300. Activity recognition model 348 may perform activity recognition to determine a probable activity associated with the motion data in the window and to estimate an activity recognition probability for each of the probable activities determined by the activity recognition model 348. Activity recognition model 348 may filter out the probable activities having associated activity recognition probabilities that are below specified confidence thresholds, and may label each of the remaining windows of motion data in free motion living data 346 with the associated probable activity having an activity recognition probability that is at or above the confidence threshold, thereby producing labeled motion data that can be incorporated into training data 341 used to train machine-learned model 300.

Note that for windows of motion data associated with a probable activity of being still having an activity recognition probability that is at or above the confidence threshold, such windows of motion data may also have to pass the stillness heuristic described above (e.g., be classified as being still), where the stillness of the window is determine based at least in part on the average accelerometer magnitude for the window in order to be labeled as being still. For example, if a window of motion data associated with a probable activity of being still has an activity recognition probability that is at or above the confidence threshold but does not pass stillness heuristic described above (e.g., is not classified as being still), training device 320 may refrain from including the window of motion data in training data 341 used to train machine-learned model 300.

In some examples, activity recognition model 348 may determine more than one probable activity from a window of motion data in free motion living data 346. For example, activity recognition model 348 may determine a probable activity for each of multiple overlapping sets of motion data, where each set of motion data includes at least some samples of motion data that are within the window of motion data. Activity recognition model 348 may therefore determine a single probable activity for the window of motion data by performing a weighted average of the multiple overlapping sets of motion data, where the weight used to perform the weighted average may correspond to the percentage of samples of motion data in a set of motion data that are inside the window of motion data.

In some examples, activity recognition model 348 may perform debouncing of windows of motion data in free motion living data 346 prior to including such windows motion data in training data 341. For example, some motion data may include random spikes of high confidence predictions that the motion data correspond to the physical activity of riding a bicycle of a few (e.g., 10) seconds in length surrounded by motion data that appear to correspond to physical activities other than cycling. Because it may be unlikely that a user would ride a bicycle for a few seconds at a time, activity recognition model 348 may perform debouncing of such windows motion data to filter out short bursts of motion data that correspond to the physical activity of riding a bicycle by taking into account the context of the surrounding windows of motion data.

Activity recognition model 348 may, for a current window having an associated probable activity of riding a bicycle, determine whether the associated probable activity of riding a bicycle for the current window is likely to be correct or not based on a configurable number of (e.g., 5) neighboring windows, such as neighboring windows to the left of the current window in free motion living data 346, the neighboring windows to the right of the current window in free motion living data 346, or the neighboring windows to the right and to the left of the current window with the current window centered between the neighboring windows. If activity recognition model 348 determines that the number of neighboring windows that are also associated with a probable activity of riding a bicycle is not greater than or equal to a specified threshold, then activity recognition model 348 may determine that the current window may be misclassified as being associated with the probable activity of riding a bicycle.

If activity recognition model 348 determines that a window may be misclassified as being associated with the probable activity of riding a bicycle, activity recognition model 348 may determine if the window may be associated with a probable activity different from riding a bicycle. For example, activity recognition model 348 may zero out (i.e., set to all zeros) the bicycling component of the activity recognition probability vector for the window and may renormalize the activity recognition probability vector for the window to 1, so that if there is another probable activity that is highly probable to being associated with the window, activity recognition model 348 may have the opportunity to associate that probable activity with the window.

In some implementations, machine-learned model 300 can be trained by optimizing an objective function, such as objective function 345. For example, in some implementations, objective function 345 may be or include a loss function that compares (e.g., determines a difference between) output data generated by the model from the training data and labels (e.g., ground-truth labels) associated with the training data. For example, the loss function can evaluate a sum or mean of squared differences between the output data and the labels. In some examples, objective function 345 may be or include a cost function that describes a cost of a certain outcome or output data. Other examples of objective function 345 can include margin-based techniques such as, for example, triplet loss or maximum-margin training.

One or more of various optimization techniques can be performed to optimize objective function 345. For example, the optimization technique(s) can minimize or maximize objective function 345. Example optimization techniques include Hessian-based techniques and gradient-based techniques, such as, for example, coordinate descent; gradient descent (e.g., stochastic gradient descent); subgradient methods; etc. Other optimization techniques include black box optimization techniques and heuristics.

In some implementations, backward propagation of errors can be used in conjunction with an optimization technique (e.g., gradient based techniques) to train machine-learned model 300 (e.g., when machine-learned model is a multi-layer model such as an artificial neural network). For example, an iterative cycle of propagation and model parameter (e.g., weights) update can be performed to train machine-learned model 300. Example backpropagation techniques include truncated backpropagation through time, Levenberg-Marquardt backpropagation, etc.

In some implementations, machine-learned model 300 described herein can be trained using unsupervised learning techniques. Unsupervised learning can include inferring a function to describe hidden structure from unlabeled data. For example, a classification or categorization may not be included in the data. Unsupervised learning techniques can be used to produce machine-learned models capable of performing clustering, anomaly detection, learning latent variable models, or other tasks.

Machine-learned model 300 can be trained using semi-supervised techniques which combine aspects of supervised learning and unsupervised learning. Machine-learned model 300 can be trained or otherwise generated through evolutionary techniques or genetic algorithms. In some implementations, machine-learned model 300 described herein can be trained using reinforcement learning. In reinforcement learning, an agent (e.g., model) can take actions in an environment and learn to maximize rewards and/or minimize penalties that result from such actions. Reinforcement learning can differ from the supervised learning problem in that correct input/output pairs are not presented, nor sub-optimal actions explicitly corrected.

In some implementations, one or more generalization techniques can be performed during training to improve the generalization of machine-learned model 300. Generalization techniques can help reduce overfitting of machine-learned model 300 to the training data. Example generalization techniques include dropout techniques; weight decay techniques; batch normalization; early stopping; subset selection; stepwise selection; etc.

In some implementations, machine-learned model 300 described herein can include or otherwise be impacted by a number of hyperparameters, such as, for example, learning rate, number of layers, number of nodes in each layer, number of leaves in a tree, number of clusters; etc. Hyperparameters can affect model performance. Hyperparameters can be hand selected or can be automatically selected through application of techniques such as, for example, grid search; black box optimization techniques (e.g., Bayesian optimization, random search, etc.); gradient-based optimization; etc. Example techniques and/or tools for performing automatic hyperparameter optimization include Hyperopt; Auto-WEKA; Spearmint; Metric Optimization Engine (MOE); etc.

In some implementations, various techniques can be used to optimize and/or adapt the learning rate when the model is trained. Example techniques and/or tools for performing learning rate optimization or adaptation include Adagrad; Adaptive Moment Estimation (ADAM); Adadelta; RMSprop; etc.

In some implementations, transfer learning techniques can be used to provide an initial model from which to begin training of machine-learned model 300 described herein.

In some implementations, machine-learned model 300 described herein can be included in different portions of computer-readable code on a computing device. In one example, machine-learned model 300 can be included in a particular application or program and used (e.g., exclusively) by such particular application or program. Thus, in one example, a computing device can include a number of applications and one or more of such applications can contain its own respective machine learning library and machine-learned model(s).

In another example, machine-learned model 300 described herein can be included in an operating system of a computing device (e.g., in a central intelligence layer of an operating system) and can be called or otherwise used by one or more applications that interact with the operating system. In some implementations, each application can communicate with the central intelligence layer (and model(s) stored therein) using an application programming interface (API) (e.g., a common, public API across all applications).

In some implementations, the central intelligence layer can communicate with a central device data layer. The central device data layer can be a centralized repository of data for the computing device. The central device data layer can communicate with a number of other components of the computing device, such as, for example, one or more sensors, a context manager, a device state component, and/or additional components. In some implementations, the central device data layer can communicate with each device component using an API (e.g., a private API).

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

In addition, the machine learning techniques described herein are readily interchangeable and combinable. Although certain example techniques have been described, many others exist and can be used in conjunction with aspects of the present disclosure.

Further to the descriptions above, a user may be provided with controls allowing the user to make an election as to both if and when systems, programs or features described herein may enable collection of user information (e.g., information about a user's social network, social actions or activities, profession, a user's preferences, or a user's current location), and if the user is sent content or communications from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the user may have control over what information is collected about the user, how that information is used, and what information is provided to the user.

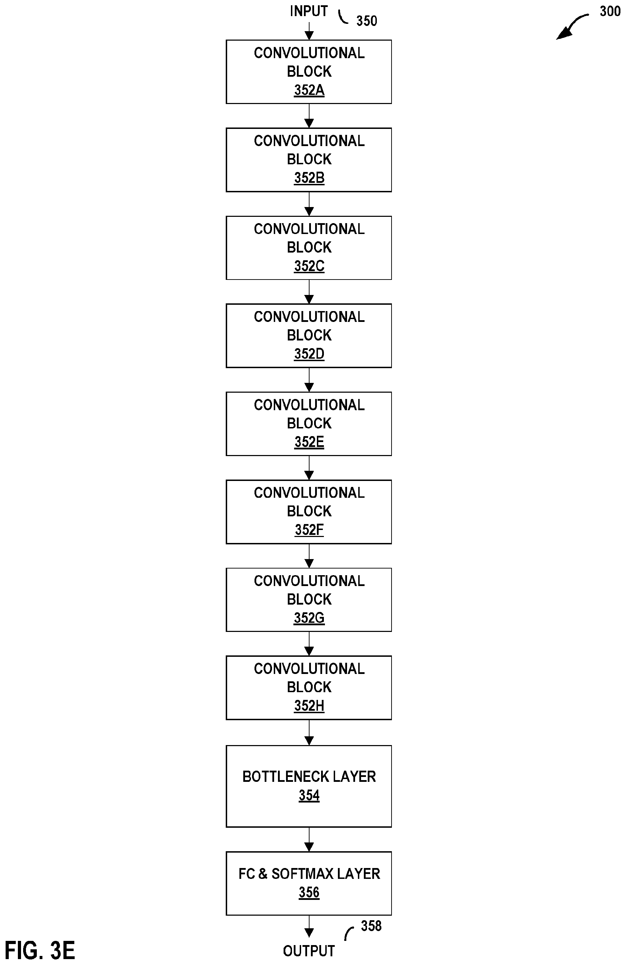

FIG. 3E illustrates a conceptual diagram illustrating an example machine-learned model. As shown in FIG. 3E, machine-learned model 300 may be a convolutional neural network that includes eight convolutional blocks 352A-352H ("convolutional blocks 352") followed by bottleneck layer 354 and SoftMax layer 356.

Each of convolutional blocks 352 may include a convolution filter followed by a rectified linear unit (ReLU) activation function and a max pooling operation. The convolution filters of convolutional blocks may have exponentially increasing capacity per layer to counteract decreasing spatial resolution from the max pooling operations. As shown in FIG. 3E, machine-learned model 300 may implement dropout regularization to reduce overfitting.

Input 350 may be 256 samples of tri-axial accelerometer data, such as sampled at 25 hertz over approximately 10 seconds. As such, the convolution filter of convolution block 352A may have a height of 256 to accept the 256 samples of input 350. Convolution filter of convolution block 352A may also have a depth of 12. Convolution block 352A may generate, from the 256-sample input 350, an output of 128 samples.

Convolution filter of convolution block 352B may have a height of 128 to accept the 128 samples outputted by convolution block 352A. Convolution filter of convolution block 352B may also have a depth of 14. Convolution block 352B may generate, from the 128 samples outputted from convolution block 352A, an output of 64 samples.

Convolution filter of convolution block 352C may have a height of 64 to accept the 64 samples outputted by convolution block 352B. Convolution filter of convolution block 352C may also have a depth of 17. Convolution block 352C may generate, from the 64 samples outputted from convolution block 352B, an output of 32 samples.

Convolution filter of convolution block 352D may have a height of 32 to accept the 32 samples outputted by convolution block 352C. Convolution filter of convolution block 352D may also have a depth of 20. Convolution block 352D may generate, from the 32 samples outputted from convolution block 352C, an output of 16 samples.

Convolution filter of convolution block 352E may have a height of 16 to accept the 16 samples outputted by convolution block 352D. Convolution filter of convolution block 352E may also have a depth of 25. Convolution block 352E may generate, from the 16 samples outputted from convolution block 352D, an output of 8 samples.

Convolution filter of convolution block 352F may have a height of 8 to accept the 8 samples outputted by convolution block 352E. Convolution filter of convolution block 352F may also have a depth of 30. Convolution block 352F may generate, from the 8 samples outputted from convolution block 352E, an output of 4 samples.

Convolution filter of convolution block 352G may have a height of 4 to accept the 4 samples outputted by convolution block 352F. Convolution filter of convolution block 352G may also have a depth of 35. Convolution block 352G may generate, from the 4 samples outputted from convolution block 352F, an output of 2 samples.

Convolution filter of convolution block 352H may have a height of 2 to accept the 2 samples outputted by convolution block 352G. Convolution filter of convolution block 352H may also have a depth of 43. Convolution block 352H may generate, from the 2 samples outputted from convolution block 352F, an output that may be fed to bottleneck layer 354.

Bottleneck layer 354 may be a 16-unit fully connected layer that flattens the output of convolutional blocks 352. Fully connected and SoftMax layer 356 may receive the output of bottleneck layer 354 to determine probabilities of the motion data of input 350 corresponding to each of a plurality of classes of physical activity. For example, fully connected and SoftMax layer 356 may determine a probability distribution, which may be floating point numbers that sum to 1, over a plurality of classes of physical activities, such as a probability distribution over the physical activities of walking, running, cycling, and being still. Fully connected and SoftMax layer 356 may therefore produce output 358 that is the probability distribution of the motion data of input 350 over a plurality of physical activities.

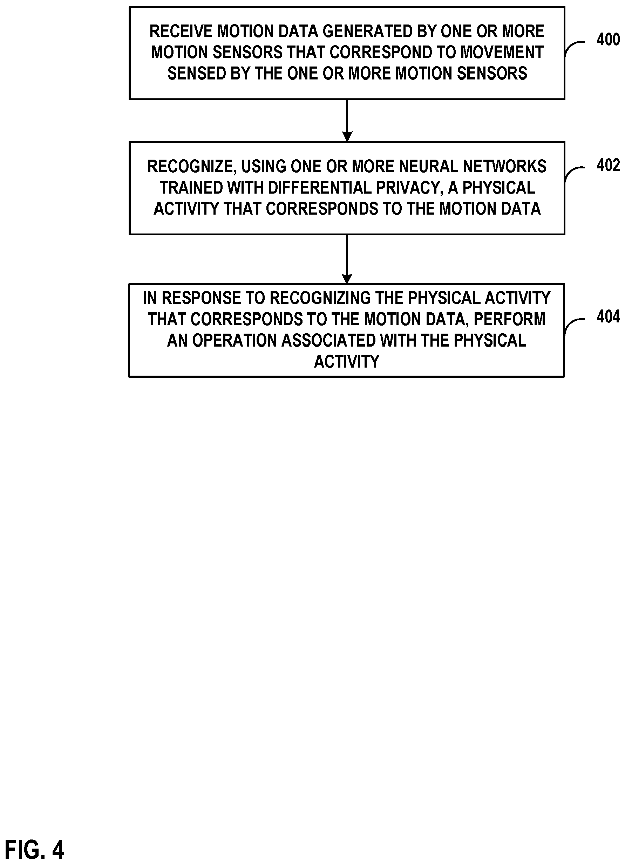

FIG. 4 is a flow diagram illustrating example operations of a computing device that may perform on-device recognition of a physical activity, in accordance with one or more aspects of the present disclosure. For purposes of illustration only, the example operations are described below within the context of computing device 110 of FIG. 1.

As shown in FIG. 4, computing device 110 may receive motion data generated by one or more motion sensors, such as one or more of sensor components 114 and/or one or more of sensor components 108, that correspond to movement sensed by the one or more motion sensors (400). Computing device 110 may perform, using one or more neural networks trained with differential privacy, on-device activity recognition to recognize a physical activity that corresponds to the motion data (402). Computing device 110 may, in response to recognizing the physical activity that corresponds to the motion data, perform an operation associated with the physical activity (406).

This disclosure includes the following examples.
Example 1: A method includes receiving, by a computing device, motion data generated by one or more motion sensors that correspond to movement sensed by the one or more motion sensors; perform, by the computing device using one or more neural networks trained with differential privacy, on-device activity recognition to recognize a physical activity that corresponds to the motion data; and in response to recognizing the physical activity that corresponds to the motion data, performing, by the computing device, an operation associated with the physical activity.
Example 2: The method of example 1, wherein the one or more neural networks are trained using a differential privacy framework and using a delta parameter that bounds a probability of a privacy guarantee of the one or more neural networks not holding, wherein the delta parameter has a value that is set to an inverse of a size of a training set for the one or more neural networks.
Example 3: The method of any of examples 1 and 2, wherein the one or more neural networks are trained using a training set of motion data corresponding to a plurality of physical activities.
Example 4: The method of example 3, wherein the training set of motion data were transformed with unconstrained random rotation in a plurality of directions.
Example 5: The method of any of examples 3 and 4, wherein the training set of motion data comprises a plurality of motion data classified as being still that were generated from users driving or sitting in a vehicle.
Example 6: The method of any of examples 3-5, wherein the training set of motion data comprises a plurality of motion data generated by a pre-trained activity recognition model based at least in part on labeling unlabeled free living motion data.
Example 7: The method of example 6, wherein the plurality of motion data generated by the pre-trained activity recognition model based at least in part on labeling the unlabeled free living motion data further was further generated by the pre-trained activity recognition model performing debouncing of a window of motion data in the unlabeled free living motion data to remove one or more short bursts of motion data that correspond to riding a bicycle based on a context of neighboring windows of motion data to the window of motion data.
Example 8: The method of any of examples 3-7, wherein the training set of motion data comprises a plurality of motion data classified as remaining still selected from unlabeled free living motion data based at least in part on average accelerometer magnitudes associated with windows of motion data in the unlabeled free living motion data.
Example 9: The method of any of examples 1-8, wherein performing the on-device activity recognition to recognize the physical activity comprises determining, using the one or more neural networks a probability distribution of the motion data across a plurality of physical activities.
Example 10: The method of any of examples 1-9, wherein receiving the motion data generated by the one or more motion sensors that correspond to the movement sensed by the one or more motion sensors further comprises: receiving, by the computing device, the motion data generated by the one or more motion sensors of a wearable computing device communicably coupled to the computing device that correspond to the movement of the wearable computing device sensed by the one or more motion sensors.
Example 11: The method of any of examples 1-10, wherein receiving the motion data generated by the one or more motion sensors that correspond to the movement sensed by the one or more motion sensors further comprises: receiving, by the computing device, the motion data generated by the one or more motion sensors of the computing device that correspond to the movement of the computing device sensed by the one or more motion sensors.
Example 12: A computing device includes: a memory; and one or more processors configured to: receive motion data generated by one or more motion sensors that correspond to movement sensed by the one or more motion sensors; perform, using one or more neural networks trained with differential privacy, on-device activity recognition to recognize a physical activity that corresponds to the motion data; and in response to recognizing the physical activity that corresponds to the motion data, perform an operation associated with the physical activity.
Example 13: The computing device of example 12, wherein the one or more neural networks are trained using a differential privacy library and using a delta parameter that bounds a probability of a privacy guarantee of the one or more neural networks not holding, wherein the delta parameter has a value that is set to an inverse of a size of a training set for the one or more neural networks.
Example 14: The computing device of any of examples 12 and 13, wherein the one or more neural networks are trained using a training set of motion data corresponding to a plurality of physical activities.
Example 15: The computing device of example 14, wherein the training set of motion data were transformed with unconstrained random rotation in a plurality of directions.
Example 16: The computing device of any of examples 14 and 15, wherein the training set of motion data comprises a plurality of motion data generated by a pre-trained activity recognition model based at least in part on labeling unlabeled free living motion data.
Example 17: A computer-readable storage medium storing instructions that, when executed, cause one or more processors of a computing device to: receive motion data generated by one or more motion sensors that correspond to movement sensed by the one or more motion sensors; perform, using one or more neural networks trained with differential privacy, on-device activity recognition to recognize a physical activity that corresponds to the motion data; and in response to recognizing the physical activity that corresponds to the motion data, perform an operation associated with the physical activity.
Example 18: The computer-readable storage medium of example 17, wherein the one or more neural networks are trained using a differential privacy library and using a delta parameter that bounds a probability of a privacy guarantee of the one or more neural networks not holding, wherein the delta parameter has a value that is set to an inverse of a size of a training set for the one or more neural networks.
Example 19: The computer-readable storage medium of any of examples 17 and 18, wherein the one or more neural networks are trained using a training set of motion data corresponding to a plurality of physical activities.
Example 20: The computer-readable storage medium of example 19, wherein the training set of motion data were transformed with unconstrained random rotation in a plurality of directions.

In one or more examples, the functions described may be implemented in hardware, software, firmware, or any combination thereof. If implemented in software, the functions may be stored on or transmitted over, as one or more instructions or code, a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media, which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Disk and disc, as used, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor," as used may refer to any of the foregoing structure or any other structure suitable for implementation of the techniques described. In addition, in some aspects, the functionality described may be provided within dedicated hardware and/or software modules. Also, the techniques could be fully implemented in one or more circuits or logic elements.

The techniques of this disclosure may be implemented in a wide variety of devices or apparatuses, including a wireless handset, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

It is to be recognized that depending on the embodiment, certain acts or events of any of the methods described herein can be performed in a different sequence, may be added, merged, or left out altogether (e.g., not all described acts or events are necessary for the practice of the method). Moreover, in certain embodiments, acts or events may be performed concurrently, e.g., through multi-threaded processing, interrupt processing, or multiple processors, rather than sequentially.

In some examples, a computer-readable storage medium includes a non-transitory medium. In some examples, the term "non-transitory" indicates that the storage medium is not embodied in a carrier wave or a propagated signal. In certain examples, a non-transitory storage medium may store data that can, over time, change (e.g., in RAM or cache). Although certain examples are described as outputting various information for display, techniques of the disclosure may output such information in other forms, such as audio, holographical, or haptic forms, to name only a few examples, in accordance with techniques of the disclosure.

Various examples have been described. These and other examples are within the scope of the following claims.

## Claims

1. A method comprising:
receiving, by a computing device, motion data generated by one or more motion sensors that correspond to movement sensed by the one or more motion sensors;
perform, by the computing device using one or more neural networks trained with differential privacy, on-device activity recognition to recognize a physical activity that corresponds to the motion data; and
in response to recognizing the physical activity that corresponds to the motion data, performing, by the computing device, an operation associated with the physical activity.

2. The method of claim 1, wherein the one or more neural networks are trained using a differential privacy framework and using a delta parameter that bounds a probability of a privacy guarantee of the one or more neural networks not holding, wherein the delta parameter has a value that is set to an inverse of a size of a training set for the one or more neural networks.

3. The method of claim 1 or claim 2, wherein the one or more neural networks are trained using a training set of motion data corresponding to a plurality of physical activities.

4. The method of claim 3, wherein the training set of motion data were transformed with unconstrained random rotation in a plurality of directions.

5. The method of claim 3 or claim 4, wherein the training set of motion data comprises a plurality of motion data classified as being still that were generated from users driving or sitting in a vehicle.

6. The method of any one of claims 3 to 5, wherein the training set of motion data comprises a plurality of motion data generated by a pre-trained activity recognition model based at least in part on labeling unlabeled free living motion data.

7. The method of claim 6, wherein the plurality of motion data generated by the pre-trained activity recognition model based at least in part on labeling the unlabeled free living motion data further was further generated by the pre-trained activity recognition model performing debouncing of a window of motion data in the unlabeled free living motion data to remove one or more short bursts of motion data that correspond to riding a bicycle based on a context of neighboring windows of motion data to the window of motion data.

8. The method of any of claims 3 to 7, wherein the training set of motion data comprises a plurality of motion data classified as remaining still selected from unlabeled free living motion data based at least in part on average accelerometer magnitudes associated with windows of motion data in the unlabeled free living motion data.

9. The method of any one of the preceding claims, wherein performing the on-device activity recognition to recognize the physical activity comprises determining, using the one or more neural networks a probability distribution of the motion data across a plurality of physical activities.

10. The method of any one of the preceding claims, wherein receiving the motion data generated by the one or more motion sensors that correspond to the movement sensed by the one or more motion sensors further comprises:
receiving, by the computing device, the motion data generated by the one or more motion sensors of a wearable computing device communicably coupled to the computing device that correspond to the movement of the wearable computing device sensed by the one or more motion sensors.

11. The method of any one of claims 1 to 9, wherein receiving the motion data generated by the one or more motion sensors that correspond to the movement sensed by the one or more motion sensors further comprises:
receiving, by the computing device, the motion data generated by the one or more motion sensors of the computing device that correspond to the movement of the computing device sensed by the one or more motion sensors.

12. A computing device includes:
a memory; and
one or more processors configured to:
receive motion data generated by one or more motion sensors that correspond to movement sensed by the one or more motion sensors;
perform, using one or more neural networks trained with differential privacy, on-device activity recognition to recognize a physical activity that corresponds to the motion data; and
in response to recognizing the physical activity that corresponds to the motion data, perform an operation associated with the physical activity.

13. The computing device of claim 12, wherein the one or more neural networks are trained using a differential privacy library and using a delta parameter that bounds a probability of a privacy guarantee of the one or more neural networks not holding, wherein the delta parameter has a value that is set to an inverse of a size of a training set for the one or more neural networks.

14. The computing device of claim 12 or claim 13, wherein the one or more neural networks are trained using a training set of motion data corresponding to a plurality of physical activities, and optionally:
wherein the training set of motion data were transformed with unconstrained random rotation in a plurality of directions and/or wherein the training set of motion data comprises a plurality of motion data generated by a pre-trained activity recognition model based at least in part on labeling unlabeled free living motion data.

15. A computer-readable storage medium storing instructions that, when executed, cause one or more processors of a computing device to perform the method of any one of claims 1 to 11.
